# EUROPEAN PATENT APPLICATION

(11) **EP 4 790 044 A1**
(43) Date of publication of application: **12.08.2026**
(21) Application number: 25869566.7
(22) Date of filing: 10.10.2025
(51) Int. Cl.: C07C 17/389, B01D 53/26, B01J 20/18, B01J 20/30, C07C 21/22

(54) **METHOD FOR PRODUCING PURIFIED FLUOROALKYNE COMPOUND, FLUORINATED ADSORBENT, AND METHOD FOR PRODUCING SAME**

(30) Priority: 20.12.2024 JP 2024225299
(71) Applicant: DAIKIN INDUSTRIES, LTD., Osaka-Shi, Osaka 530-0001 (JP)
(72) Inventor: ETO, Yusuke, Osaka-shi, Osaka 530-0001 (JP); NAKAMURA, Shingo, Osaka-shi, Osaka 530-0001 (JP); KUROKI, Yoshichika, Osaka-shi, Osaka 530-0001 (JP); SUGIYAMA, Akinari, Osaka-shi, Osaka 530-0001 (JP)
(74) Representative: Hoffmann Eitle
(86) International application number: PCT/JP2025/036002
(87) International publication number: WO 2026/133698

(57) **Abstract**

Provided is a method for producing a purified fluoroalkyne compound, wherein the method includes subjecting a water-containing fluoroalkyne compound to a dehydration process using a fluorinated adsorbent to obtain a purified fluoroalkyne compound, and the fluoroalkyne compound is a fluoroalkyne compound represented by the formula 1.

## Description

### TECHNICAL FIELD

The present disclosure is related to a method for producing a purified fluoroalkyne compound, a fluorinated adsorbent, and a method for producing thereof.

### BACKGROUND ART

Conventionally, as a purification method for a fluorinated hydrocarbon compound such as hexafluorobutadiene, octafluorocyclobutane, hexafluorobutadiene, and octafluorocyclopentene, a method of using an adsorbent for dehydration process to purify these fluorinated hydrocarbon compounds has been known (PTLs 1 to 3). A zeolite and boron oxide are known as an adsorbent used for dehydration process (PTLs 1 to 3).

### CITATION LIST

### PATENT LITERATURE

PTL 1: U.S. Patent No. 6544319
PTL 2: Korean Patent Publication No. 2023-0134179
PTL 3: WO 2007/063938

### SUMMARY OF INVENTION

### TECHNICAL PROBLEM

Conventionally, a fluoroalkyne compound that is a hydrofluorocarbon has been used in a dry etching gas for a semiconductor, various refrigerants, a foaming agent, a heat transfer medium, and the like. The study has revealed that if an adsorbent is used as it is in dehydration process as a purification method of a fluoroalkyne compound, the adsorbent and the fluoroalkyne compound react causing a fluoroalkyne compound-derived organic compound other than the fluoroalkyne compound to be easily formed, and it becomes difficult to obtain a highly pure fluoroalkyne compound.

The present disclosure is based on the aforementioned circumstances and aims to provide a method for producing a purified fluoroalkyne compound, a fluorinated adsorbent, and a method for producing thereof that allow to suppress the formation of a fluoroalkyne compound-derived organic compound other than the fluoroalkyne compound in the dehydration process of the fluoroalkyne compound.

### SOLUTION TO PROBLEM

The present disclosure comprises the following constitutions.

[Item 1] A method for producing a purified fluoroalkyne compound including subjecting a water-containing fluoroalkyne compound to a dehydration process using a fluorinated adsorbent to obtain a purified fluoroalkyne compound, wherein the fluoroalkyne compound is a compound represented by the formula 1:
[Chem. 1]

R¹-C≡C-R² formula 1

wherein, R¹ and R² each independently represent a hydrogen atom, a fluorine atom, or a fluoroalkyl group; and at least one of R¹ and R² represents the fluoroalkyl group.

[Item 2] The method for producing the purified fluoroalkyne compound according to item 1 wherein the fluorinated adsorbent has a percentage content of fluorine atoms of 1 atm% or more.

[Item 3] The method for producing the purified fluoroalkyne compound according to item 1 or 2 wherein the fluorinated adsorbent is a fluorinated zeolite adsorbent.

[Item 4] The method for producing the purified fluoroalkyne compound according to any one of items 1 to 3 wherein the fluoroalkyl group is a CF₃ group, a C₂F₅ group, a C₃F₇ group, or a C₄F₉ group.

[Item 5] A method for producing a fluorinated adsorbent including treating a surface of an adsorbent with a fluorinating agent.

[Item 6] The method for producing the fluorinated adsorbent according to item 5 wherein the adsorbent is a zeolite adsorbent.

[Item 7] The method for producing the fluorinated adsorbent according to item 5 or 6 wherein the fluorinating agent is at least one type selected from the group consisting of hydrogen fluoride, fluorine, chlorodifluoromethane, dichlorofluoromethane, and a fluoroalkyne compound wherein the fluoroalkyne compound is a compound represented by the formula 2:
[Chem. 2]

**R³-C≡C-R⁴** formula 2

wherein, R³ and R⁴ each independently represent a hydrogen atom, a fluorine atom, or a fluoroalkyl group; and at least one of R³ and R⁴ represents the fluoroalkyl group.

[Item 8] The method for producing the fluorinated adsorbent according to item 7 wherein the fluoroalkyl group is a CF₃ group, a C₂F₅ group, a C₃F₇ group, or a C₄F₉ group.

[Item 9] A fluorinated adsorbent for dehydration of a fluoroalkyne compound wherein the fluoroalkyne compound is a compound represented by the formula 1:
[Chem. 3]

R¹-C≡C-R² formula 1

wherein, R¹ and R² each independently represent a hydrogen atom, a fluorine atom, or a fluoroalkyl group; and at least one of R¹ and R² represents the fluoroalkyl group.

[Item 10] The fluorinated adsorbent according to item 9 wherein the fluorinated adsorbent has a percentage content of fluorine atoms of 1 atm% or more.

[Item 11] The fluorinated adsorbent according to item 9 or 10 wherein the fluorinated adsorbent is a fluorinated zeolite adsorbent.

[Item 12] The fluorinated adsorbent according to any one of items 9 to 11 wherein the fluoroalkyl group is a CF₃ group, a C₂F₅ group, a C₃F₇ group, or a C₄F₉ group.

### ADVANTAGEOUS EFFECTS OF INVENTION

The present disclosure can provide a method for producing a purified fluoroalkyne compound, a fluorinated adsorbent, and a method for producing thereof that allow to suppress the formation of a fluoroalkyne compound-derived organic compound other than the fluoroalkyne compound in the dehydration process of the fluoroalkyne compound.

### DESCRIPTION OF EMBODIMENTS

Specific examples for the method for producing the purified fluoroalkyne compound, the fluorinated adsorbent, and the method for producing thereof as one embodiment of the present disclosure (hereinafter also referred to as the "present embodiment") are explained below.

In the present specification, an expression "A to B" indicates a range of upper and lower limits (that is, A or more and B or less), and if no unit is mentioned for A but a unit is mentioned only for B, the units for A and B are the same.

In the present disclosure, if a compound or the like is expressed by a chemical formula without limiting the atomic ratio, it includes various atomic ratio known in the art and is not necessarily restricted to those within the stoichiometric range.

### [Embodiment 1: Method for producing a purified fluoroalkyne compound]

A method for producing a purified fluoroalkyne compound according to one embodiment of the present disclosure is explained.

One embodiment of the present disclosure (hereinafter also referred to as the "present embodiment") is a method for producing a purified fluoroalkyne compound including subjecting a water-containing fluoroalkyne compound to a dehydration process using a fluorinated adsorbent to obtain a purified fluoroalkyne compound, wherein the fluoroalkyne compound is a compound represented by the formula 1:
[Chem. 4]

R¹-C≡C-R² formula 1

wherein, R¹ and R² each independently represent a hydrogen atom, a fluorine atom, or a fluoroalkyl group; and at least one of R¹ and R² represents the fluoroalkyl group.

The present disclosure can provide a method for producing a purified fluoroalkyne compound that allows to suppress the formation of a fluoroalkyne compound-derived organic compound other than the fluoroalkyne compound in the dehydration process of the fluoroalkyne compound. The reasons are presumed to be as below.
(a) If a fluoroalkyne compound that contains water and is represented by the formula 1 is subjected to a dehydration process using an adsorbent as it is, the adsorbent and the fluoroalkyne compound react causing a fluoroalkyne compound-derived organic compound (for example, C₄F₇H if the fluoroalkyne compound is hexafluoro-2-butyne) other than the fluoroalkyne compound to be easily formed.
(b) In the method for producing the purified fluoroalkyne compound according to the present embodiment, the fluoroalkyne compound is subjected to a dehydration process using a fluorinated adsorbent. As a result, the activity of the active sites on the adsorbent can be suppressed, and a fluoroalkyne compound-derived organic compound other than the fluoroalkyne compound is less likely to be formed.

### <<Obtaining a purified fluoroalkyne compound>>

The method for producing the purified fluoroalkyne compound according to the present disclosure includes subjecting a water-containing fluoroalkyne compound to a dehydration process using a fluorinated adsorbent to obtain a purified fluoroalkyne compound (hereinafter also simply referred to as "obtaining a purified fluoroalkyne compound"). The phrase "includes "obtaining a purified fluoroalkyne compound"" means that the method may only include "obtaining a purified fluoroalkyne compound", or it may include an additional step in addition to "obtaining a purified fluoroalkyne compound". An example of the "additional step" includes "obtaining a fluorinated adsorbent" later described.

For "obtaining a purified fluoroalkyne compound", "subjecting a water-containing fluoroalkyne compound to a dehydration process using a fluorinated adsorbent" (hereinafter also simply referred to as "dehydration process") may be implemented in the liquid phase or the gas phase. The dehydration process implemented in the liquid phase means subjecting the "water-containing fluoroalkyne compound" in the liquid state to a dehydration process. The dehydration process implemented in the gas phase means subjecting the "water-containing fluoroalkyne compound" in the gas state to a dehydration process. If the dehydration process is implemented in the liquid phase, the dehydration process may be implemented in a batch type or a flow type (continuous type), but the dehydration process is preferably implemented in a batch type. If the dehydration process is implemented in the gas phase, the dehydration process may be implemented in a batch type or a flow type (continuous type), but the dehydration process is preferably implemented in a flow type (continuous type).

For "obtaining a purified fluoroalkyne compound", the parts by mass of the fluorinated adsorbent is preferably 0.01 parts by mass or more and 200 parts by mass or less based on 100 parts by mass of the water-containing fluoroalkyne compound. As a result, it becomes easier to suppress the formation of a fluoroalkyne compound-derived organic compound other than the fluoroalkyne compound in the dehydration process of the fluoroalkyne compound. The parts by mass of the fluorinated adsorbent is more preferably 0.05 parts by mass or more and 150 parts by mass or less, even more preferably 0.1 parts by mass or more and 100 parts by mass or less based on 100 parts by mass of the water-containing fluoroalkyne compound.

A temperature of the dehydration process, if implemented in the liquid phase, is preferably -50°C or more and 100°C or less. As a result, it becomes easier to suppress the formation of a fluoroalkyne compound-derived organic compound other than the fluoroalkyne compound in the dehydration process of the fluoroalkyne compound. The temperature is more preferably -20°C or more and 75°C or less, even more preferably 0°C or more and 50°C or less.

The temperature of the dehydration process, if implemented in the gas phase, is preferably -10°C or more and 100°C or less. As a result, it becomes easier to suppress the formation of a fluoroalkyne compound-derived organic compound other than the fluoroalkyne compound in the dehydration process of the fluoroalkyne compound. The temperature is more preferably 0°C or more and 75°C or less, even more preferably 10°C or more and 50°C or less.

The pressure of the dehydration process, if implemented in the liquid phase, is preferably -30 kPaG or more and 3,200 kPaG or less in gauge pressure. As a result, it becomes easier to suppress the formation of a fluoroalkyne compound-derived organic compound other than the fluoroalkyne compound in the dehydration process of the fluoroalkyne compound. The pressure is more preferably 0 kPaG or more and 2,000 kPaG or less, even more preferably 100 kPaG or more and 1,100 kPaG or less, in gauge pressure.

The pressure of the dehydration process, if implemented in the gas phase, is preferably 0 kPaG or more and 3,000 kPaG or less in gauge pressure. As a result, it becomes easier to suppress the formation of a fluoroalkyne compound-derived organic compound other than the fluoroalkyne compound in the dehydration process of the fluoroalkyne compound. The pressure is more preferably 10 kPaG or more and 2,000 kPaG or less, even more preferably 20 kPaG or more and 1,500 kPaG or less, in gauge pressure.

The duration of the dehydration process (in other words, the time during which the water-containing fluoroalkyne compound is in contact with the fluorinated adsorbent) is preferably 0.01 seconds or more and 6,000 seconds or less. As a result, it becomes easier to suppress the formation of a fluoroalkyne compound-derived organic compound other than the fluoroalkyne compound in the dehydration process of the fluoroalkyne compound. The duration is more preferably 0.1 seconds or more and 3,000 seconds or less, even more preferably 1 second or more and 1,800 seconds or less.

### <<Fluoroalkyne compound>>

The fluoroalkyne compound is represented by the formula 1:
[Chem. 5]

**R¹-C≡C-R²** formula 1

wherein, R¹ and R² each independently represent a hydrogen atom, a fluorine atom, or a fluoroalkyl group; and at least one of R¹ and R² represents the fluoroalkyl group.

In the formula 1, the number of carbon atoms in the fluoroalkyl group is preferably 1 or more and 4 or less. As a result, it becomes easier to suppress the formation of a fluoroalkyne compound-derived organic compound other than the fluoroalkyne compound in the dehydration process of the fluoroalkyne compound. In the formula 1, the number of carbon atoms in the fluoroalkyl group may be 1, 2, 3, or 4.

In the formula 1, the fluoroalkyl group is preferably a perfluoroalkyl group. As a result, it becomes easier to suppress the formation of a fluoroalkyne compound-derived organic compound other than the fluoroalkyne compound in the dehydration process of the fluoroalkyne compound.

In the formula 1, the fluoroalkyl group is preferably a CF₃ group, a C₂F₅ group, a C₃F₇ group, or a C₄F₉ group. As a result, it becomes easier to suppress the formation of a fluoroalkyne compound-derived organic compound other than the fluoroalkyne compound in the dehydration process of the fluoroalkyne compound. The C₃F₇ group may be linear or branched. The C₃F₇ group may be -CF₂CF₂CF₃ or - CF(CF₃)₂. The C₄F₉ group may be linear or branched. The C₄F₉ group may be - (CF₂)₃CF₃, -CF₂CF(CF₃)₂, -CF(CF₃)CF₂CF₃, or -C(CF₃)₃.

In the formula 1, R¹ and R² are both preferably fluoroalkyl groups. As a result, it becomes easier to suppress the formation of a fluoroalkyne compound-derived organic compound other than the fluoroalkyne compound in the dehydration process of the fluoroalkyne compound.

Examples the fluoroalkyne compound represented by the formula 1 include CF₃C≡CCF₃, CF₃C≡CCF₂CF₃, CF₃C≡CCF(CF₃)₂, CF₃C≡CC(CF₃)₃, CF₃CF₂C≡CCF₂CF₃, CF₃CF₂C≡CCF(CF₃)₂, CF₃CF₂C≡CC(CF₃)₃, (CF₃)₂CFC≡CCF(CF₃)₂, (CF₃)₂CFC≡CC(CF₃)₃, and (CF₃)₃CC≡CC(CF₃)₃, CF≡CCF₃, CF≡CCF₂CF₃, CF≡CCF(CF₃)₂, and CF≡CC(CF₃)₃.

The water-containing fluoroalkyne compound according to the present disclosure preferably has a percentage content of water of 0.001 vol ppm or more and 500 vol ppm or less. As a result, it becomes easier to suppress the formation of a fluoroalkyne compound-derived organic compound other than the fluoroalkyne compound in the dehydration process of the fluoroalkyne compound. The percentage content of water is more preferably 0.005 vol ppm or more and 400 vol ppm or less, even more preferably 0.01 vol ppm or more and 300 vol ppm or less.

The percentage content of water can be measured by using a quartz crystal moisture measuring meter.

The water-containing fluoroalkyne compound according to the present disclosure may be prepared by synthesizing a fluoroalkyne compound using a conventionally known method, or it may be prepared by purchasing a commercially available product.

### <<Fluorinated adsorbent>>

### <Formulation>

The fluorinated adsorbent according to the present disclosure has a percentage content of fluorine atoms of preferably 1 atm% or more. As a result, a fluoroalkyne compound-derived organic compound other than the fluoroalkyne compound is less likely formed when the water-containing fluoroalkyne compound is subjected to the dehydration process using a fluorinated adsorbent. The lower limit of the percentage content of fluorine atoms in the fluorinated adsorbent is more preferably 1.5 atm% or more, even more preferably 2 atm% or more. The upper limit of the percentage content of fluorine atoms in the fluorinated adsorbent is not limited, but for example, the upper limit is preferably 10 atm% or less, more preferably 8 atm% or less, even more preferably 5 atm% or less. The percentage content of fluorine atoms in the fluorinated adsorbent is preferably 1 atm% or more and 10 atm% or more, more preferably 1.5 atm% or more and 8 atm% or more, even more preferably 2 atm% or more and 5 atm% or more. It should be noted that in the present disclosure, an adsorbent means a substance which adsorbs at least water.

The percentage content of fluorine atoms in the fluorinated adsorbent can be identified by using an X-ray photoelectron spectroscopy (XPS).

### <Types>

The fluorinated adsorbent may be at least one type selected from the group consisting of, for example, a fluorinated zeolite adsorbent, a fluorinated activated carbon adsorbent, a fluorinated alumina adsorbent, and a fluorinated silica-alumina. The fluorinated adsorbent is preferably a fluorinated zeolite adsorbent. Especially, as aluminum oxide (Al₂O₃) contained in the zeolite adsorbent is fluorinated, the activity of the active sites on the zeolite adsorbent is more easily suppressed, and so it becomes easier to suppress the formation of a fluoroalkyne compound-derived organic compound other than the fluoroalkyne compound in the dehydration process of the fluoroalkyne compound. It should be noted that for similar reasons, the adsorbent is preferably a zeolite adsorbent for "treating the surface of the adsorbent with a fluorinating agent" later described.

Whether the fluorinated adsorbent is a fluorinated zeolite adsorbent can be identified by XPS or an energy dispersion x-ray spectroscopy (EDX). The same applies for whether the adsorbent is a zeolite adsorbent for "treating the surface of the adsorbent with a fluorinating agent" later described.

The fluorinated zeolite adsorbent may contain a cation. The cation is not limited, but examples include H⁺, Li⁺, Na⁺, K⁺, Ca²⁺, Mg²⁺, and Ba²⁺. From the viewpoint of suppressing the decomposition of a compound, the cation is preferably at least one type selected from the group consisting of Na⁺, K⁺, Ca²⁺, and Mg²⁺. The same applies to when the adsorbent (in other words, the adsorbent before being fluorinated) is a zeolite adsorbent for "treating the surface of the adsorbent with a fluorinating agent" later described.

A type of the cation in the fluorinated zeolite adsorbent can be identified by an ion exchange chromatography. It should be noted that when "treating the surface of the adsorbent with a fluorinating agent" later described, if the adsorbent is a zeolite adsorbent, the type of the cation in the zeolite adsorbent can be identified by the same method as the method for identifying the type of the cation in the "fluorinated zeolite adsorbent", except that the target to measure is a "zeolite adsorbent" (in other words, a "zeolite adsorbent" before being fluorinated).

A zeolite composing the fluorinated zeolite adsorbent may be a synthetic zeolite or a natural zeolite, but preferably it is a synthetic zeolite such as a molecular sieve.

The ratio N2/N1 of the number of silicon atoms N2 to the number of aluminum atoms N1 in the fluorinated zeolite adsorbent, is preferably 0.1 or more and 20 and less. As a result, it becomes easier to suppress the formation of a fluoroalkyne compound-derived organic compound other than the fluoroalkyne compound in the dehydration process of the fluoroalkyne compound. The ratio N2/N1 is more preferably 1 or more and 10 or less, even more preferably 2 or more and 6 or less.

The ratio N2/N1 in the fluorinated zeolite adsorbent can be identified by XPS or EDX.

### <Pore diameter>

The fluorinated adsorbent has pores, and a pore diameter of the pores is preferably 1 Å or more and 20 Å or less. As a result, it becomes easier to suppress the formation of a fluoroalkyne compound-derived organic compound other than the fluoroalkyne compound in the dehydration process of the fluoroalkyne compound. The pore diameter of the fluorinated adsorbent is more preferably 2 Å or more and 15 Å or less, even more preferably 3 Å or more and 10 Å or less.

The pore diameter can be identified by using a specific surface area and pore diameter distribution measuring instrument.

The fluorinated adsorbent may also be produced by "obtaining the fluorinated adsorbent" as an additional step. "Obtaining the fluorinated adsorbent" may include "treating the surface of the adsorbent with a fluorinating agent".

### <Treating the surface of the adsorbent with a fluorinating agent>

For "treating the surface of the adsorbent with a fluorinating agent", the reaction of the adsorbent and the fluorinating agent may be implemented in the liquid phase or the gas phase. If the reaction is implemented in the liquid phase, a reaction format of the adsorbent and the fluorinating agent may be a batch type or a flow type (continuous type), but the reaction format is preferably a batch type. If the reaction is implemented in the gas phase, a reaction format of the adsorbent and the fluorinating agent may be a batch type or a flow type (continuous type), but the reaction format is preferably a flow type.

In the "reaction of the adsorbent and the fluorinating agent", the parts by mass of the fluorinating agent is preferably 0.01 parts by mass or more and 10,000 parts by mass or less based on 100 parts by mass of the adsorbent. As a result, a fluorinated adsorbent is more easily formed, and it becomes easier to suppress the formation of a fluoroalkyne compound-derived organic compound other than the fluoroalkyne compound in the dehydration process of the fluoroalkyne compound. The parts by mass of the fluorinating agent is more preferably 0.1 parts by mass or more and 5,000 parts by mass or less, even more preferably 1 part by mass or more and 3,000 parts by mass or less, based on 100 parts by mass of the adsorbent.

The temperature of the "reaction of the adsorbent and the fluorinating agent", if implemented in the liquid phase, is preferably -50°C or more and 100°C or less. As a result, a fluorinated adsorbent is more easily formed, and it becomes easier to suppress the formation of a fluoroalkyne compound-derived organic compound other than the fluoroalkyne compound in the dehydration process of the fluoroalkyne compound. The temperature is more preferably -20°C or more and 75°C or less, even more preferably 0°C or more and 50°C or less.

The temperature of the "reaction of the adsorbent and the fluorinating agent", if implemented in the gas phase, is preferably 10°C or more and 500°C or less. As a result, a fluorinated adsorbent is more easily formed, and it becomes easier to suppress the formation of a fluoroalkyne compound-derived organic compound other than the fluoroalkyne compound in the dehydration process of the fluoroalkyne compound. The temperature is more preferably 15°C or more and 450°C or less, even more preferably 20°C or more and 400°C or less.

The pressure of the "reaction of the adsorbent and the fluorinating agent", if implemented in the liquid phase, is preferably -80 kPaG or more and 3,000 kPaG or less in gauge pressure. As a result, a fluorinated adsorbent is more easily formed, and it becomes easier to suppress the formation of a fluoroalkyne compound-derived organic compound other than the fluoroalkyne compound in the dehydration process of the fluoroalkyne compound. The pressure is more preferably -50 kPaG or more and 2,500 kPaG or less, even more preferably 0 kPaG or more and 2,000 kPaG or less, in gauge pressure.

The pressure of the "reaction of the adsorbent and the fluorinating agent", if implemented in the gas phase, is preferably 0 kPaG or more and 3,000 kPaG or less in gauge pressure. As a result, a fluorinated adsorbent is more easily formed, and it becomes easier to suppress the formation of a fluoroalkyne compound-derived organic compound other than the fluoroalkyne compound in the dehydration process of the fluoroalkyne compound. The pressure is more preferably 5 kPaG or more and 2,500 kPaG or less, even more preferably 10 kPaG or more and 2,000 kPaG or less, in gauge pressure.

The duration of the "reaction of the adsorbent and the fluorinating agent" (in other words, the time during which the adsorbent is in contact with the fluorinating agent) is preferably 0.01 seconds or more and 6,000 seconds or less. As a result, a fluorinated adsorbent is more easily formed, and it becomes easier to suppress the formation of a fluoroalkyne compound-derived organic compound other than the fluoroalkyne compound in the dehydration process of the fluoroalkyne compound. The duration is more preferably 0.1 seconds or more and 3,000 seconds or less, even more preferably 1 second or more and 1,800 seconds or less.

For "treating the surface of the adsorbent with a fluorinating agent", the fluorinating agent is at least one type selected from the group consisting of hydrogen fluoride, fluorine, chlorodifluoromethane, dichlorofluoromethane, and a fluoroalkyne compound wherein the fluoroalkyne compound is preferably a compound represented by the formula 2:
[Chem. 6]

**R³-C≡C-R⁴** formula 2

wherein, R³ and R⁴ each independently represent a hydrogen atom, a fluorine atom, or a fluoroalkyl group; and at least one of R³ and R⁴ represents the fluoroalkyl group.

The number of carbon atoms in the fluoroalkyl group in the formula 2 is preferably 1 or more and 4 or less. As a result, it becomes easier to suppress the formation of a fluoroalkyne compound-derived organic compound other than the fluoroalkyne compound in the dehydration process of the fluoroalkyne compound. The number of carbon atoms in the fluoroalkyl group in the formula 2 may be 1, 2, 3, or 4.

The fluoroalkyl group in the formula 2 is preferably a perfluoroalkyl group. As a result, it becomes easier to suppress the formation of a fluoroalkyne compound-derived organic compound other than the fluoroalkyne compound in the dehydration process of the fluoroalkyne compound.

In the formula 2, the fluoroalkyl group is preferably a CF₃ group, a C₂F₅ group, a C₃F₇ group, or a C₄F₉ group. As a result, it becomes easier to suppress the formation of a fluoroalkyne compound-derived organic compound other than the fluoroalkyne compound in the dehydration process of the fluoroalkyne compound. The C₃F₇ group may be linear or branched. The C₃F₇ group may be -CF₂CF₂CF₃ or - CF(CF₃)₂. The C₄F₉ group may be linear or branched. The C₄F₉ group may be - (CF₂)₃CF₃, -CF₂CF(CF₃)₂, -CF(CF₃)CF₂CF₃, or -C(CF₃)₃.

In the formula 2, R³ and R⁴ are both preferably fluoroalkyl groups. As a result, it becomes easier to suppress the formation of a fluoroalkyne compound-derived organic compound other than the fluoroalkyne compound in the dehydration process of the fluoroalkyne compound.

Examples of the fluoroalkyne compound represented by the formula 2 include CF₃C≡CCF₃, CF₃C≡CCF₂CF₃, CF₃C≡CCF(CF₃)₂, CF₃C≡CC(CF₃)₃, CF₃CF₂C≡CCF₂CF₃, CF₃CF₂C≡CCF(CF₃)₂, CF₃CF₂C≡CC(CF₃)₃, (CF₃)₂CFC≡CCF(CF₃)₂, (CF₃)₂CFC≡CC(CF₃)₃, and (CF₃)₃CC≡CC(CF₃)₃, CF≡CCF₃, CF≡CCF₂CF₃, CF≡CCF(CF₃)₂, and CF≡CC(CF₃)₃.

From the viewpoint of the efficiency in producing the purified fluoroalkyne compound according to Embodiment 1, the fluoroalkyne compound represented by the formula 2 is preferably the same compound as the fluoroalkyne compound represented by the formula 1.

### <Applications>

The purified fluoroalkyne compound obtained by the method for producing the purified fluoroalkyne compound according to the present embodiment can be effectively used in applications such as an etching gas. The etching gas is useful for forming cutting-edge microstructures of a semiconductor, liquid crystal, and the like.

### [Embodiment 2: Method for producing a fluorinated adsorbent]

A method for producing a fluorinated adsorbent according to the present embodiment is explained.

The present embodiment is a method for producing a fluorinated adsorbent including treating the surface of the adsorbent with a fluorinating agent.

The present disclosure can provide a method for producing a fluorinated adsorbent that allows to suppress the formation of a fluoroalkyne compound-derived organic compound other than the fluoroalkyne compound in the dehydration process of the fluoroalkyne compound. The reasons are presumed to be as described in Embodiments 1(a) and (b).

### <<Treating the surface of the adsorbent with a fluorinating agent>>

"Treating the surface of the adsorbent with a fluorinating agent" according to the present embodiment is the same as "treating the surface of the adsorbent with a fluorinating agent" according to Embodiment 1, and so the description thereof is omitted.

### <Applications>

The fluorinated adsorbent obtained by the method for producing the fluorinated adsorbent according to the present embodiment can be effectively used in applications such as dehydration of a water-containing fluoroalkyne compound.

### [Embodiment 3: Fluorinated adsorbent]

The fluorinated adsorbent according to the present embodiment is explained.

The present embodiment is a fluorinated adsorbent for dehydration of a fluoroalkyne compound wherein the fluoroalkyne compound is represented by the formula 1:
[Chem. 7]

**R¹-C≡C-R²** formula 1

wherein, R¹ and R² each independently represent a hydrogen atom, a fluorine atom, or a fluoroalkyl group; and at least one of R¹ and R² represents the fluoroalkyl group.

The present disclosure can provide a fluorinated adsorbent that allows to suppress the formation of a fluoroalkyne compound-derived organic compound other than the fluoroalkyne compound in the dehydration process of the fluoroalkyne compound. The reasons are presumed to be as described in Embodiments 1(a) and (b).

### <<Fluorinated adsorbent>>

The fluorinated adsorbent according to the present embodiment is the same as the fluorinated adsorbent according to Embodiment 1, and so the description thereof is omitted.

### <<Fluoroalkyne compound>>

The fluoroalkyne compound according to the present embodiment is the same as the fluoroalkyne compound according to Embodiment 1, and so the description thereof is omitted.

### <Applications>

The fluorinated adsorbent according to the present embodiment is a fluorinated adsorbent for dehydration of a fluoroalkyne compound.

### <<Method for producing a fluorinated adsorbent>>

The fluorinated adsorbent according to the present embodiment can be produced by, for example, the method for producing the fluorinated adsorbent described in Embodiment 2.

The embodiments of the present disclosure were described above, but various modifications are possible in the embodiments and details without departing from the purpose and scope of the claims.

### [Examples]

The present embodiments are described more specifically with reference to examples. However, the present embodiments are not limited to these examples.

### <<Preparation of adsorbent>>

The adsorbents of samples 1 to 8 and samples 101 to 104 were prepared as below.

Firstly, a metal tube made of stainless steel (diameter of 1/2 inch, length of 30 cm) was filled with a molecular sieve stated in Table 1 (pore diameter: as stated in Table 1; ratio N2/N1 of the number of silicone atoms, N2, to the number of aluminum atoms, N1: as stated in Table 1; cation contained in the zeolite adsorbent: as stated in Table 1) as a zeolite adsorbent with the amount stated in Table 1.

Then, the metal tube was depressurized to a pressure stated in Table 1 using a pump. Then, for the cases where the numerical number of more than "0" is stated in the column for "filling amount for fluorinating agent [g]" in Table 1, the metal tube was filled with an amount stated in Table 1 of hexafluoro-2-butyne (fluorinating agent) until the pressure inside the metal tube reached normal pressure at room temperature and was left to stand for the duration stated in Table 1 to fluorinate the molecular sieve stated in Table 1 (in other words, zeolite adsorbent). The adsorbent of each sample was prepared as above.

### <<Characterization of adsorbent>>

### <Percentage content of fluorine atoms in the adsorbent>

A percentage content [atm%] of fluorine atoms in the adsorbent of each sample was measured by the method described in Embodiment 1. The obtained results are listed in the column "percentage content of fluorine atoms [atm%]" in Table 2.

### <<Production of purified fluoroalkyne compound>>

The metal tube filled with the above adsorbent of each sample was circulated with hexafluoro-2-butyne (fluoroalkyne compound) having a percentage contents of water of 52 vol ppm and C₄F₇H of below detection limit, with a flow rate of 300 ccm for 30 minutes at room temperature to obtain a purified fluoroalkyne compound.

### <Characterization of purified fluoroalkyne compound>

### <Water>

The percentage content of water in the purified fluoroalkyne compound was measured by using a quartz crystal moisture measuring meter. The obtained results are listed in the column "percentage content of water [vol ppm]" in Table 2.

### <C₄F₇H>

The percentage content of C₄F₇H (in other words, the fluoroalkyne compound-derived organic compound other than the fluoroalkyne compound) in the purified fluoroalkyne compound was measured by a gas chromatography. The obtained results are listed in the column "percentage content of C₄F₇H [vol ppm]" in Table 2. It should be noted that "ND (not detectable)" means that the value was below detection limit and specifically indicates that the value was "less than 0.1 vol ppm".

If the percentage content of C₄F₇H is below detection limit in the purified fluoroalkyne compound, this indicates that the formation of a fluoroalkyne compound-derived organic compound other than the fluoroalkyne compound was suppressed in the dehydration process of the fluoroalkyne compound.

### [Table 1]

**Table 1**

| Sample No. | Adsorbent (before being fluorinated) | | | | Pressure [kPaG] | Filling amount of fluorinating agent [g] | Duration [minutes] |
|---|---|---|---|---|---|---|---|
| | Cation | Pore diameter [Å] | N2/N1 | Filling amount [g] | | | |
| 1 | K⁺ | 3 | 2 | 5.0 | 500 | 1.3 | 20 |
| 2 | Na⁺ | 4 | 2 | 5.0 | 500 | 1.3 | 20 |
| 3 | Ca²⁺ | 5 | 2 | 5.0 | 500 | 1.3 | 20 |
| 4 | Na⁺, K⁺, Ca²⁺,Mg²⁺ | 5 | 6 | 5.0 | 500 | 1.3 | 20 |
| 5 | K⁺ | 3 | 2 | 5.0 | 500 | 1.3 | 90 |
| 6 | Na⁺ | 4 | 2 | 5.0 | 500 | 1.3 | 90 |
| 7 | Ca²⁺ | 5 | 2 | 5.0 | 500 | 1.3 | 90 |
| 8 | Na⁺, K⁺, Ca²⁺,Mg²⁺ | 5 | 6 | 5.0 | 500 | 1.3 | 90 |
| 101 | K⁺ | 3 | 2 | 5.0 | 500 | - | - |
| 102 | Na⁺ | 4 | 2 | 5.0 | 500 | - | - |
| 103 | Ca²⁺ | 5 | 2 | 5.0 | 500 | - | - |
| 104 | Na⁺, K⁺, Ca²⁺,Mg²⁺ | 5 | 6 | 5.0 | 500 | - | - |

### [Table 2]

**Table 2**

| Sample No. | Adsorbent | Purified fluoroalkyne compound | |
|---|---|---|---|
| | Percentage content of fluorine atoms [atm%] | Percentage content of water [vol ppm] | Percentage content of C₄F₇H [vol ppm] |
| 1 | 1 | 0.1 | ND |
| 2 | 1 | 0.1 | ND |
| 3 | 1 | 0.1 | ND |
| 4 | 1 | 0.1 | ND |
| 5 | 5 | 0.1 | ND |
| 6 | 5 | 0.1 | ND |
| 7 | 5 | 0.1 | ND |
| 8 | 5 | 0.1 | ND |
| 101 | 0 | 0.1 | 10 |
| 102 | 0 | 0.1 | 11 |
| 103 | 0 | 0.1 | 9 |
| 104 | 0 | 0.1 | 10 |

The methods for producing the purified fluoroalkyne compounds for samples 1 to 8 correspond to Examples. The methods for producing the fluoroalkyne compounds for samples 101 to 104 correspond to Comparative Examples. It was observed that the method for producing the purified fluoroalkyne compounds for samples 1 to 8 exhibited exceptional effects of being able to suppress the formation of the fluoroalkyne compound-derived organic compound other than the fluoroalkyne compound in the dehydration process of the fluoroalkyne compound in comparison to the method for producing the purified fluoroalkyne compounds for samples 101 to 104. Therefore, it was found that the method for producing the purified fluoroalkyne compounds for samples 1 to 8 can suppress the formation of the fluoroalkyne compound-derived organic compound other than the fluoroalkyne compound in the dehydration process of the fluoroalkyne compound.

## Claims

1. A method for producing a purified fluoroalkyne compound comprising subjecting a water-containing fluoroalkyne compound to a dehydration process using a fluorinated adsorbent to obtain a purified fluoroalkyne compound, wherein
the fluoroalkyne compound is a compound represented by the formula 1:
[Chem. 1]
**R¹-C≡C-R²** formula 1
wherein, R¹ and R² each independently represent a hydrogen atom, a fluorine atom, or a fluoroalkyl group; and at least one of R¹ and R² represents the fluoroalkyl group.

2. The method for producing the purified fluoroalkyne compound according to claim 1 wherein the fluorinated adsorbent has a percentage content of fluorine atoms of 1 atm% or more.

3. The method for producing the purified fluoroalkyne compound according to claim 1 or 2 wherein the fluorinated adsorbent is a fluorinated zeolite adsorbent.

4. The method for producing the purified fluoroalkyne compound according to claim 1 or 2 wherein the fluoroalkyl group is a CF₃ group, a C₂F₅ group, a C₃F₇ group, or a C₄F₉ group.

5. A method for producing a fluorinated adsorbent comprising treating a surface of an adsorbent with a fluorinating agent.

6. The method for producing the fluorinated adsorbent according to claim 5 wherein the adsorbent is a zeolite adsorbent.

7. The method for producing the fluorinated adsorbent according to claim 5 or 6 wherein the fluorinating agent is at least one type selected from the group consisting of hydrogen fluoride, fluorine, chlorodifluoromethane, dichlorofluoromethane, and a fluoroalkyne compound, wherein
the fluoroalkyne compound is a compound represented by the formula 2:
[Chem. 2]
**R³-C≡C-R⁴** formula 2
wherein, R³ and R⁴ each independently represent a hydrogen atom, a fluorine atom, or a fluoroalkyl group; and at least one of R³ and R⁴ represents the fluoroalkyl group.

8. The method for producing the fluorinated adsorbent according to claim 7 wherein the fluoroalkyl group is a CF₃ group, a C₂F₅ group, a C₃F₇ group, or a C₄F₉ group.

9. A fluorinated adsorbent for dehydration of a fluoroalkyne compound, wherein
the fluoroalkyne compound is a compound represented by the formula 1:
[Chem. 3]
**R¹-C≡C-R²** formula 1
wherein, R¹ and R² each independently represent a hydrogen atom, a fluorine atom, or a fluoroalkyl group; and at least one of R¹ and R² represents the fluoroalkyl group.

10. The fluorinated adsorbent according to claim 9 wherein the fluorinated adsorbent has a percentage content of fluorine atoms of 1 atm% or more.

11. The fluorinated adsorbent according to claim 9 or 10 wherein the fluorinated adsorbent is a fluorinated zeolite adsorbent.

12. The fluorinated adsorbent according to claim 9 or 10 wherein the fluoroalkyl group is a CF₃ group, a C₂F₅ group, a C₃F₇ group, or a C₄F₉ group.
